# EUROPEAN PATENT SPECIFICATION

(11) **EP 2 379 549 B1**
(45) Date of publication and mention of the grant of the patent: **21.09.2016**
(21) Application number: 09795936.5
(22) Date of filing: 21.12.2009
(51) Int. Cl.: C07D 471/04, C07D 519/00, A61K 31/437, A61P 35/00, A61P 25/28, A61P 31/12

(54) **7-AZAINDIRUBINS, 7'-AZAINDIRUBINS, 7-7'-DIAZAINDIRUBIN AND THE CORRESPONDING 3'-OXIME ETHER DERIVATES: PRODUCTION THEREOF, THEIR PRODUCTION AND USE AS A MEDICAMENT**
7-AZAINDIRUBINE, 7'-AZAINRUBINE, 7-7'-DIAZAINRUBIN UND ENTSPRECHENDE 3'-OXIMETHERDERIVATE: HERSTELLUNG DAVON, HERSTELLUNG UND VERWENDUNG ALS MEDIKAMENT
7-AZAINDIRUBINS, 7'-AZAINDIRUBINS, 7-7'-DIAZAINDIRUBIN ET LES DÉRIVÉS D'ÉTHER DE 3'-OXIME CORRESPONDANTS : PRODUCTION CORRESPONDANTE, LEUR PRODUCTION ET UTILISATION EN TANT QUE MÉDICAMENT

(30) Priority: 22.12.2008 EP 08022288; 30.06.2009 EP 09008544
(43) Date of publication of application: 26.10.2011
(73) Proprietor: Eisenbrand, Gerhard, 69126 Heidelberg (DE)
(72) Inventor: MERZ, Karl-Heinz, 67098 Bad Dürkheim (DE); CHENG, XinLai, 67655 Kaiserslautern (DE)
(74) Representative: Müller-Boré & Partner Patentanwälte PartG mbB
(86) International application number: PCT/EP2009/009202
(87) International publication number: WO 2010/072399

(56) References cited:
- WO-A-2005/041954
- DATABASE CA [Online] CHEMICAL ABSTRACTS SERVICE, COLUMBUS, OHIO, US; 2007, YAO, QIZHENG ET AL: "Method for synthesis of 7-azo-indigo red and 7-azo-isoindigotin derivatives and their medicinal application" XP002523996 retrieved from STN Database accession no. 2007:1345845 & CN 101 074 229 A (WUXI JIEXI PHARMACEUTICAL SCIENCE AND TECHNOLOGY CO., LTD., PEOP. REP.) 21 November 2007 (2007-11-21)

## Description

The present invention relates to 7'-azaindirubins (2) and 7,7'-diaza-indirubins (3), wherein E and R² have the meanings detailed in the description, their production and use as a medicament for treating cancer, neurodegenerative diseases, bipolar disorders, inflammatory and infectious diseases, including viral diseases.

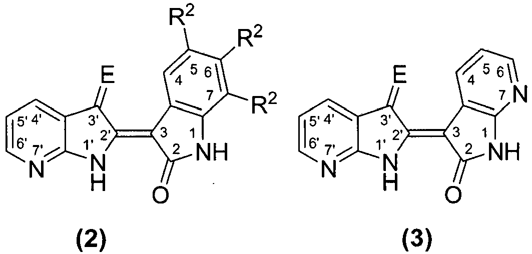

Depending on structure, these indirubins act as inhibitors of various kinases involved in tumor cell growth, and, most notably, as inhibitors of human tumor cell proliferation. As compared to indirubins bearing identical substituents but no hetero atoms in position 7 and 7', the activity of the compounds according to the present invention is increased and the propensity to get metabolized by CYP450s is reduced, resulting in improved metabolic stability.

In traditional Chinese medicine (TCM), indigo naturalis, therein known as "qing dai", is mentioned as integral part of remedies with hemostatic, antipyretic, anti-inflammatory, antileukaemic, sedative, antibacterial, and antiviral properties. The reported antileukaemic activity was found to be associated with the minor ingredient indirubin, a 3,2'-bisindole isomer of indigo. Long-term studies in animals showed indirubin to be well tolerable and devoid of bone marrow toxicity or hematotoxicity. Indirubin was found clinically to be active against chronic myeloic leukemia (CML) in China. Early studies, aimed at elucidating the mechanism of action, reported inhibition of DNA and protein synthesis.

In 1999, indirubins were discovered to potently inhibit cyclin dependent kinases (CDKs), competing with ATP at the ATP binding site, and to effectively inhibit the growth of various human tumor cell lines in the low micromolar range (Hössel et al. 1999, Nature Cell Biol 1, 60-67). Moreover, later on certain indirubin derivatives were found to interfere with an array of cancer associated cellular targets in addition to CDKs, including glycogen synthase kinase 3ß (GSK- 3ß) (Leclerc et al. 2001, J. Biol. Chem. 276, 251-260) but also receptor- and non-receptor tyrosine kinases, most prominently VEGFRs and c-Src (Jautelat et al. 2005, ChemBioChem 6, 531-540; Nam et al. 2005, Proc. Natl. Acad. Sci. U S A. 102, 5998-6003). Furthermore, glycogen phosphorylase b regulating glucose/glycogen homeostasis also was found to be affected. It also was reported that certain indirubins can interact with the aryl hydrocarbon receptor (AhR), also known as the dioxin receptor. Upon binding to the xenobiotic-responsive element (XRE), activated AhR induces the transcription of numerous genes, including cytochrome P450 CYP1A1, p27kip1, etc. (Elferink, 2003, in: Progr Cell Cycle Res, vol. 5, (eds. Meijer L. et al.), 261-267). Finally, 7-bromoindirubin-3'-oxime has been shown to trigger the activation of non-apoptotic cell death (Ribas et al., 2006, Oncogene, 1-15).

The pharmacological action of several basic indirubin derivatives is described in WO 99/62503, and tumor cell growth inhibition data of a panel of substituted indirubins have been disclosed in WO 00/61555.

Synthesis and antitumor proliferative activity of 7-azaindirubins, substituted with an alkyl chain or a sugar moiety in 1-position have been disclosed in the Chinese patent application CN 101074229A. Therein, 1-substituted-7-azaisatins required for condensation with indoxyl acetates were produced by oxidation of 1-substituted-7-azaindole using chromium(VI) oxide. However, 1-unsubstituted 7-azaindirubins are unknown as yet. These compounds cannot be prepared by the synthetic approach utilized for N-substituted 7-azaindoles. Neither their synthesis nor their activity has been reported as yet. Thus, even though in the Chinese patent application CN 101074229A, in the table, first row, on page 13 thereof 7-azaindirubin is indicated, the Chinese inventors could not have had this compound. An oxidation of 7-azaindole by chromium(VI) oxide does not lead to 7-azaisatin, but black tar. Not any experimental data are given for said compound in the table, first row, on page 13 of the Chinese patent application CN 101074229A. However, a disclosure is relevant only if the teaching it contains is reproducible. This required reproducibility, however, is not applicable for said compound given in the table, first row, on page 13 of CN 101074229A.

Incubations of 5-methylindirubin with liver microsomes have been shown to produce mainly two metabolites, 6-hydroxy-5-methyl-indirubin and 6,7'-dihydroxy-5-methylindirubin (see WO 03/070703). Whereas the first metabolite proved to be as effective as the parent compound for inhibition of murine C6 glioblastoma cells, the latter was completely inactive in inhibiting proliferation of LXFL529L tumor cells (human large cell lung tumor xenograft). Therefore, CYP mediated metabolism, especially by generating 7'-hydroxylated metabolites, is considered responsible for this decrease in antitumor efficacy.

It is therefore desirable to obtain compounds with enhanced metabolic stability towards CYP enzymes which are predominantly, yet not exclusively expressed in the liver.

The solution to the above technical problem is achieved by the embodiments characterized in the claims.

In particular, the present invention relates to 7'-azaindirubins (2), and 7,7'-diaza-indirubins **(3),** wherein E and R² have the meanings detailed in the description, their production and use as a medicament for treating cancer, neurodegenerative diseases, bipolar disorders, and infectious diseases. Blocking positions of indirubin derivatives prone to such metabolic hydroxylation should contribute to safeguard antitumor activity. Introduction of electron attracting heteroatoms into aromatic carbocycles is known to result in a decreased electron density in the heterocycle. This has been expected to impede metabolic oxidative transformation by CYP450s.

Indirubins generally are poorly soluble in nearly all solvents, presumably as a consequence of intermolecular forces that stabilize supermolecular aggregates.

For increased water solubility and bioavailability, indirubins bearing hydrophilic substituents in the 3'-oxime ether and the 5-carboxamide position were synthesized (Merz & Eisenbrand (2006) in: Indirubin, the red shade of indigo, ed. L. Meijer et al., Life in Progress editions; Merz at al. 2007, Targets Oncol. 2, S46). In particular, substituents with primary, secondary or tertiary amino groups, (easily to be transformed into physiologically compatible salts) connected via an alkyl spacer to the 3'-oxime ether position, were shown to increase water solubility by orders of magnitude (data comprehensively published at the EORTC-PAMM Meeting in Berlin, 2007). For enhanced water solubility and bioavailability of 7-aza-indirubins (1), 7'-aza-indirubins (2), and 7,7'-diaza-indirubins (3), such substituents are useful as well.

It has now been found that 7'-azaindirubins (2), and 7,7'-diazaindirubins (3) exhibit excellent properties for treatment of cancer, neurodegenerative diseases, bipolar disorders, inflammatory and infectious diseases, including viral diseases. 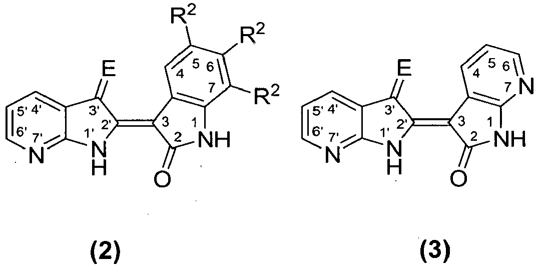 In formula (2) or (3),
R² stands for hydrogen, C₁-C₄ alkyl, halogen, hydroxy, C₁-C₄ alkoxy, mercapto, carboxy, or an amino group NR⁴R⁵, or a carboxamido group CONR⁴R⁵, wherein R⁴ and R⁵ can be the same or different and represent a hydrogen atom, a straight-chain or branched-chain C₁-C₄ alkyl group,
   which can additionally carry one or more halogen, hydroxy and/or amino groups, a substituted or unsubstituted aryl group which can comprise one or more heteroatoms, or the acyl groups -COM, -COOM, -CH₂COOM wherein M is hydrogen, a straight-chain or branched-chain alkyl group having 1 to 4 carbon atoms which can additionally carry one or more hydroxy and/or amino groups, or an aryl group which can comprise one or more heteroatoms and can be substituted with one or more halogen atoms, one or more alkyl groups or one or more alkoxy groups;
or C₃-C₇ cycloalkyl, C₃-C₇ cycloalkenyl,
   that is optionally interrupted by one or more oxygen atoms and/or can additionally carry one or more C₁-C₄ alkyl, halogen, hydroxy and/or amino groups, a substituted or unsubstituted aryl group which can comprise one or more heteroatoms, or the acyl groups -COM, -COOM, -CH₂COOM with the definition for M above,
or R⁴ and R⁵ together with the nitrogen atom of the amino group form a ring,
   which can contain one or more heteroatoms and have 2 to 8, optionally substituted, CH₂ groups which can additionally carry one or more C₁-C₄ alkyl, C₃-C₇ cycloalkyl, halogen, hydroxy and/or amino groups, a substituted or unsubstituted aryl group which can comprise one or more heteroatoms, or the acyl groups -COM, -COOM, -CH₂COOM with the definition for M above;
E in formulae (2) and (3) means an oxygen atom, an oxime group [NOH], or an oxime ether group [NOR³], with R³ representing an alkyl chain with formula (CH₂)ₙR', in which n stands for 2-6 and R' stands for an amino group NR⁴R⁵, wherein R⁴ and R⁵ can be the same or different and represent a hydrogen atom, a straight-chain or branched-chain C₁-C₄ alkyl group,
   which can additionally carry one or more halogen, hydroxy and/or amino groups, a substituted or unsubstituted aryl group which can comprise one or more heteroatoms, or the acyl groups -COM, -COOM, -CH₂COOM wherein M is hydrogen, a straight-chain or branched-chain alkyl group having 1 to 4 carbon atoms which can additionally carry one or more hydroxy and/or amino groups, or an aryl group which can comprise one or more heteroatoms and can be substituted with one or more halogen atoms, one or more alkyl groups or one or more alkoxy groups;
or C₃-C₇ cycloalkyl, C₃-C₇ cycloalkenyl,
   that is optionally interrupted by one or more oxygen atoms and/or can additionally carry one or more C₁-C₄ alkyl, halogen, hydroxy and/or amino groups, a substituted or unsubstituted aryl group which can comprise one or more heteroatoms, or the acyl groups -COM, -COOM, -CH₂COOM with the definition for M above,
or R⁴ and R⁵ together with the nitrogen atom of the amino group form a ring,
   which can contain one or more heteroatoms and have 2 to 8, optionally substituted, CH₂ groups which can additionally carry one or more C₁-C₄ alkyl, C₃-C₇ cycloalkyl, halogen, hydroxy and/or amino groups, a substituted or unsubstituted aryl group which can comprise one or more heteroatoms, or the acyl groups -COM, -COOM, -CH₂COOM with the definition for M above.

If a basic group is included, the physiologically compatible salts of organic and inorganic acids, such as hydrochloric acid, sulfuric acid, phosphoric acid, citric acid, tartaric acid, succinic acid, and others, are suitable, representing highly water soluble compounds. Transformation of the amino compounds into these salts is easily to be performed by chemists skilled in the art.

Most effective are compounds of formula (2), or (3), in which E stands for C=O or for C=NO(CH₂)ₙNR⁴R⁵, wherein n is 2, and NR⁴R⁵ represents an N-methylpiperazino substituent, as well as the acid salts thereof.

Prevention of the above mentioned deactivating metabolic transformation by CYP450 mediated introduction of a hydroxy group into the 7'-position is achieved by replacing the 7'-carbon ring atom with a 7'-nitrogen ring atom. Thus, incubation of 5-methyl-7'-aza-indirubin with liver microsomes produced 5-hydroxymethyl- and 5-methyl-6-hydroxy-7'-aza-indirubin as main metabolites, since the preferential 7'-hydroxylation, as observed with the 7'-carbon ring atom analogue was blocked. Likewise, introduction of a nitrogen atom in position 7 will hamper metabolic transformation in the unprimed part of the molecule.

The antiproliferative activity of 7'-azaindirubin against human cancer cell lines was found markedly augmented, as compared to the parent molecule, indirubin.

In the course of the present invention it has now surprisingly been found that the introduction of an additional nitrogen atom into 7-position of 7'-azaindirubin, or into 7'-position of 7-azaindirubin, leading to 7,7'-diazaindirubin, is further and rather substantially enhancing antiproliferative activity towards tumor cells such as human non small cell lung cancer cells LXFL529L. This increase in antiproliferative effectiveness corresponds to a factor of about 100, as compared to 7'-azaindirubin.

The extraordinarily high activity of this compound is evident from inhibition of proliferation of HT29 colon tumor cells and MCF-7 breast cancer cells with IC50 values ranging down to 2 nM. Table 1 summarizes data of antiproliferative activity.

The antiproliferative activity was determined using the Sulforhodamin B assay (SRB assay) as reported previously (Vatter et al. 2005, Int. J. Clin. Pharmacol. Ther. 43, 607*).*

**Table 1: Inhibition of tumor cell proliferation, SRB assay.**

| Cpd | 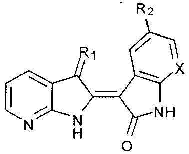 | | | Inhibition of cell proliferation IC₅₀ [µM] | | |
|---|---|---|---|---|---|---|
| | X | R₁ | R₂ | LXFL529L | HT29 | MCF7 |
| E843 | C | O | H | 4.4 | | |
| E844 | C | O | CH₃ | 4.0 | | |
| E846 | C | 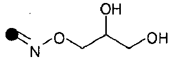 | H | 3.5 | | |
| E847 | C | O | OCH₃ | 3.6 | | |
| E864 | N | O | H | 0.07 | 0.005 | 0.007 |
| E865 | C | 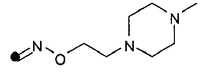 | H | 0.8 | | |

To use the compounds according to the invention as pharmaceutical agents, they may be brought into the form of a pharmaceutical preparation in the usual manner, which in addition to the active ingredient contains pharmaceutical, organic or inorganic support media according to the state of the art.

The synthesis of the compounds according to the invention is performed via condensation of an 7-azaisatin with 3-indoxyl-3-acetate or 7-aza-3-indoxyl-3-acetate by basic or acid reaction conditions, affording 7'aza-indirubin **(2)** or 7,7'-diazaindirubin **(3),** respectively. 1-Acetylindoxyl or 1,3-diacetylindoxyl, or the corresponding 7-aza-indoxyl derivatives may serve as partner for the condensation reactions as well.

The synthesis of the compounds according to formulae (2) and (3) in accordance with the present invention and compounds according to formula (1) (not in accordance with the present invention) is exemplified for the highly effective compounds as depicted in scheme 1:
Condensation of isatin (5a) or 7-azaisatin (5b) with 3-indoxyl-3-acetate (4a) or 7-aza-3-indoxyl-3-acetate (4b) is achieved by basic or acid reaction conditions, affording 7-azaindirubin (1), 7'aza-indirubin (2) or 7,7'-diazaindirubin (3), respectively. The 7-azaindiribuin (1) is a reference compound.

In position 5, 6, and/or 7-substituted isatins or in position 4, 5, 6, and/or 7 substituted 3-indoxyl-3-acetates may serve as partner for the condensation reactions as well. These methods are based on syntheses of indirubins previously reported by Russell & Kaupp, (1969), JACS 91, 3851-59, and Jautelat et al. (2005), ChemBioChem, 6, 531 -540.

Substituted isatins and indoxyl components for the coupling reactions may be produced by procedures published in literature, e.g. Holt & Sadler (1958), Proc. R. Soc. London (B) 148, 481-94; Friedländer et al. (1912), Liebigs Ann. Chem. 388, 23-49; Sandmeyer (1919), Helv Chim Acta 2, 234-42; Hewawasam & Meanwell (1994), Tetrahedron Lett. 35, 7303-6.

Reaction of the aza- or diazaindirubins **1, 2,** or 3 with hydroxylamine hydrochloride in refluxing pyridine produces 7-azaindirubin-3'-oxime **(6),** 7'-azaindirubin-3'-oxime **(7),** or 7,7'-diazaindirubin **(8),** respectively. This method is based on the synthesis of indirubin-3'-oximes reported previously (Farbwerke 1913, DE 283726).

The corresponding 3'-oxime 2-bromoethyl ethers **(9), (10),** or **(11)** are prepared by alkylation of the oximes using 1,2-dibromoethane in ethanol and tetramethyl guanidine (TMG). This method is based on the synthesis of indirubin-3'-oxime alkyl ethers previously reported by Merz & Eisenbrand (2006) in: Indirubin, the red shade of indigo, ed. L. Meijer et al., Life in Progress editions.

The 3'-oxime 2-bromoethyl ethers **(9), (10),** or **(11)** can easily be converted into more water soluble derivatives, such as the corresponding 2-(4-methylpiperazino)-ethyl ethers **(12), (13),** and **(14),** for example, by reaction in DMF with an excess of N-methyl piperazine at room temperature. This method is based on the synthesis of indirubin-3'-oxime alkyl ethers previously reported by Ferandin et al. 2006, J. Med. Chem. 49, 4638-4649.

The steps of the synthesis are outlined in scheme 1. For simplification, the substituents R¹ and R² are neglected.

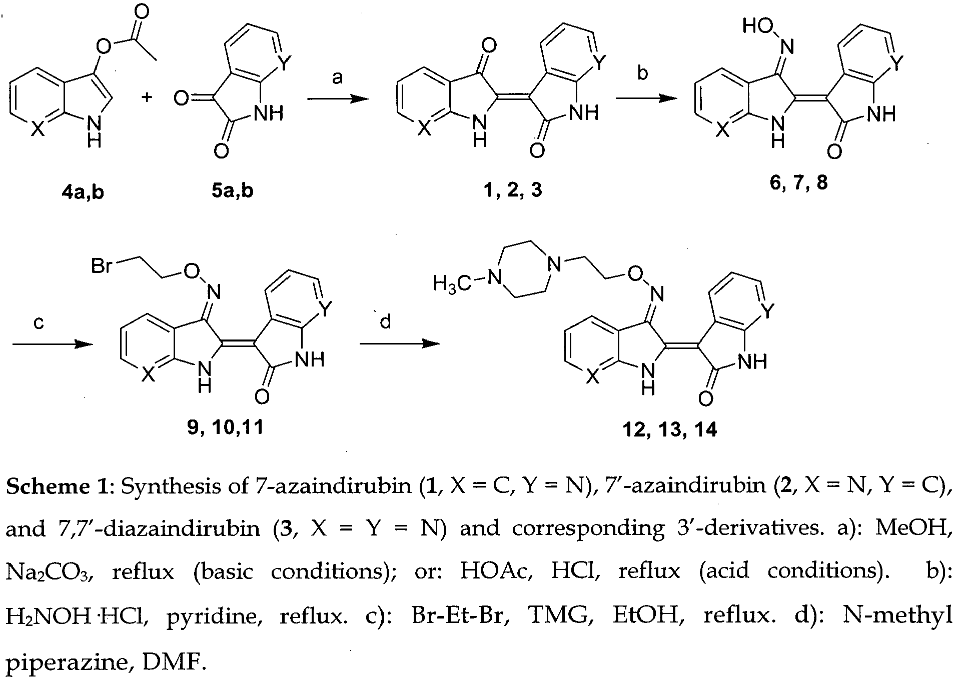

A further object of the present invention is the production of 7'-azaindirubin (**2**) or 7,7'-diaza-indirubin (**3**) by acid or basic condensation of 1-acetyl-7-aza-3-indolinone **(17),** which can be used instead of 7-aza-3-indoxyl-3-acetate (**4b**), for acid or basic condensations with isatin (**5a**) or 7-azaisatin (**5b**).

1-Acetyl-7-aza-3-indolinone **(17)** is synthesized in about 10 % overall yield by the following route (scheme 2):

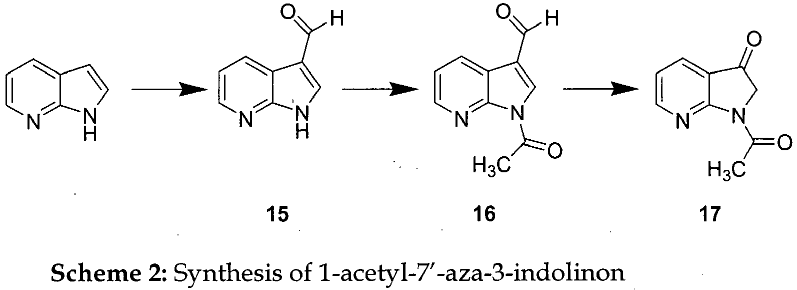

Reflux of commercially available 7-azaindole and hexamethylenetetramine in 33% acetic acid yields 3-formyl-7-azaindole **(15)** (Verbiscar et al. 1972, J. Med. Chem. 15, 149). Acetylation of **(15)** with acetic anhydride in presence of triethylamine and DMAP affords 1-acetyl-3-formyl-7-azaindole **(16).** Baeyer-Villiger oxidation of **(16)** in dichloromethane using 3-chloroperoxybenzoic acid affords 1-acetyl-7-aza-3-indolinone **(17)** (Desarbre et al., 1994, Tetrahedron Lett. 35, 1995-1998).

For condensation reactions with 1-acetyl-7-aza-3-indolinone, 5-, 6- or 7-substituted isatins, e.g. 5-methylisatin **(5c),** are applicable as well (see scheme 3). The acid or basic conditions for condensation depend on the substituents attached to the isatin core.

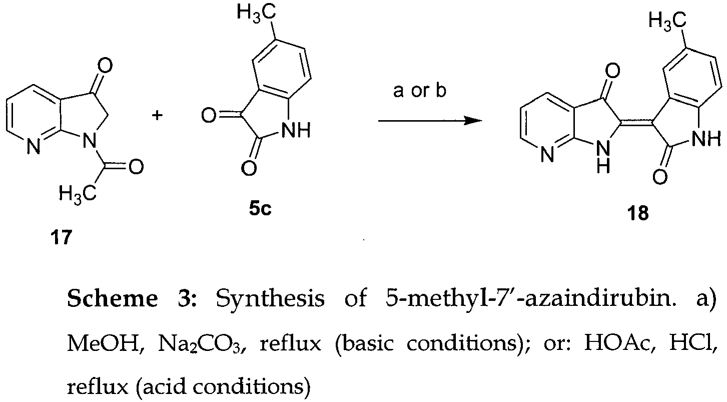

Another important object in the course of the present invention is the synthesis of 7-aza-3-indoxyl acetate (**4b**), which is produced by the reaction of 7-azaindole using thallium(III) acetate (1.1 eq.) in acetic acid / water (5%) under reflux. The oxidation provided the product in a yield up to 32% (see scheme 4). This method is based on the oxidation of indole for the synthesis of 3-acetoxyindole previously reported by Banerji et al. 1998, J. Indian Chem. Soc. 75, 698-704.

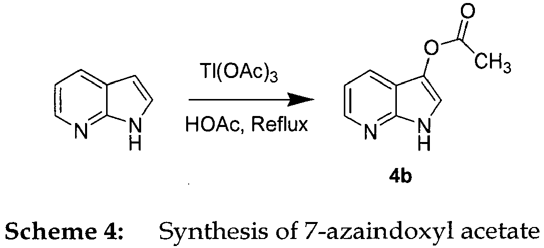

A further object in the course of the present invention is the synthesis of 7-azaindirubin, to be accomplished by acid or basic condensation of 4-, 5-, 6- and/or 7-substituted or unsubstituted 3-indoxyl-3-acetate, 1-acetyl-3-indoxyl or 1,3-diacetylindoxyl with 7-azaisatin. The novel synthesis of 7-azaisatin **(5b)** by oxidation of 7-aza-oxindole with N-bromosuccinimide and DMSO (see scheme 5) has been a key object in the course of this invention.

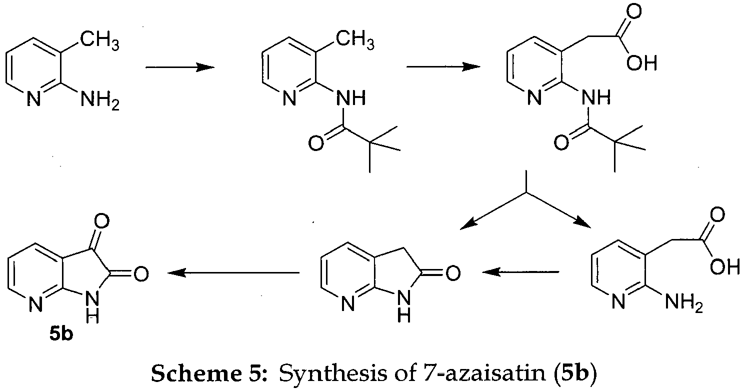

Commercially available 2-amino-3-picoline is protected with a pivaloyl group, lithiated and reacted with carbon dioxide to yield 7-azaoxindole either by hydrolysis with 3 N HCl and concomitant cyclization (Scherlock & Tom, 1991, US5023265) or by hydrolysis with 6 N HCl and subsequent cyclization in amyl alcohol by means of 4-toluene sulfonic acid (Ting et al., 1990, J. Med. Chem. 33, 2697-2706).

Previously reported syntheses of 7-azaisatins, e.g. by oxidation of 7-azaindoles using indium-(III)-chloride and 2-iodoxybenzoic acid (Yadav et al., 2007, Synthesis, 693-696), or by oxidation of 1-alkyl-7-azaindole using N-bromosuccinimide and DMSO (Tatsugi et al. 2001, ARKIVOC, 67-73) comprise only methods for the synthesis of 1-substituted 7-aza-isatins.

The method for the synthesis of 1-unsubstituted 7-azaisatin reported by Kaegi 1941, (Helv. Chim. Acta, 24, 151E-150E), is a time-consuming, cumbersome procedure with low yield.

A further object in the course of this invention is the synthesis of 7,7'-diaza-indirubin **(3)** generated as the main reaction product, when 7-azaindole (1 eq.) is transformed with thallium(III)-acetate (0.55 eq.) in glacial acetic acid at 90 °C in absence of water. Separation of **(3)** from 7-azaindoxyl acetate **(4b),** which is produced as minor byproduct during this oxidation process, is achieved by recrystallization using ethyl acetate.

The following examples will point out the syntheses that are objects of this invention.

### Example 1: 7-Azaindoxyl-3-acetate

Under argon atmosphere at room temperature, a solution of thallium(III) acetate (3.82 g, 10.0 mmol) in acetic acid (51 mL) and water (2.5 mL) was added dropwise to a solution of 7-azaindole (1.075 g, 9.1 mmol) in glacial acetic acid (7.5 mL). The mixture was refluxed for 1 h, poured onto crushed ice (200 g) and neutralized with sodium carbonate at 0°C to yield 7-azaindoxyl-3-acetate (454 mg, 2.6 mmol, 28.7%) as a reddish solid.
¹H-NMR (400 MHz, DMSO-d₆, δ = 2.49 ppm): 11.58 (s, 1H, NH), 8.24 (dd, 1H, ³J_{H,H} = 4.4 Hz, ⁴J_{H,H} = 1.3 Hz, 6-CH), 7.83 (dd, 1 H, ³J_{H,H} = 8.3 Hz, ⁴J_{H,H} = 1.8 Hz, 4-CH), 7.42 (s, 1 H, 2-CH), 7.07 (dd, 1 H, ³J_{H,H} = 4.4 Hz, ³J_{H,H} = 7.9 Hz, 5-CH), 2.31 (s, 3H, CH₃)
¹³C-{¹H}-NMR (150 MHz, DMSO-d₆, δ = 39.5 ppm): 168.7 (C=O), 144.9 (C3a), 143.3 (C6), 127.2 (C7a), 125.7 (C4), 115.4 (C2 or C5), 115.0 (C2 or C5), 112.3 (C3), 20.4 (OCH₃).

### Example 2: 7'-Azaindirubin (Method 1)

Under argon atmosphere at room temperature, a suspension of 7-azaindoxyl-3-acetate (200 mg, 1.1 mmol), isatin (183 mg, 1.2 mmol) and sodium carbonate (264 mg, 2.5 mmol) in degassed methanol (25 mL) was stirred for 5 h. The reddish precipitate was filtered off, washed with methanol and water and dried to afford 7'-azaindirubin (251 mg, 1 mmol, 87%).
¹H-NMR (600 MHz, DMSO-d₆, δ = 2.49 ppm): 11.02 (s, 1H, N'H), 10.78 (s, 1H, NH), 8.68 (d, 1H, ³J_{H,H} = 7.7 Hz, 4-CH), 8.48 (d, 1 H, ³J_{H,H} = 3.5 Hz, 6'-CH), 8.10 (d, 1 H, ³J_{H,H} = 6.2 Hz, 4'-CH), 7.29 (t, 1 H, ³J_{H,H} = 7.6 Hz, 6-CH), 7.11 (dd, 1 H, ³J_{H,H} = 5.0 Hz, ³J_{H,H} = 7.3 Hz, 5'-CH), 7.04 (t, 1 H, ³J_{H,H} = 7.6 Hz, 5-CH), 6.92 (d, 1 H, ³J_{H,H} = 7.6 Hz, 7-CH).
¹³C-{¹H}-NMR (150 MHz, DMSO-d₆, δ = 39.5 ppm): 186.1 (C3'), 171.1 (C2), 162.9 (C7a'), 155.7 (C6), 141.4 (C7a), 137.6 (C2'), 133.6 (C4'), 130.2 (C4), 124.9 (C6), 121.6 (C5), 119.2 (C3a), 117.8 (C5'), 112.9 (C3a'), 110.0 (C7), 108.2 (C3).

### Example 3: 7, 7'-Diazaindirubin (Method 1)

Under argon atmosphere at 90°C, a solution of thallium(III) acetate (1.91 g, 5 mmol) in glacial acetic acid (25 mL) was added dropwise within 2 h to a solution of 7-azaindol (1.075 g, 9.1 mmol) in glacial acetic acid (7.5 mL). The mixture was stirred overnight at 90 °C, cooled with ice and the solvent was removed by evaporation. The residue was extracted with ethyl acetate, filtrated and the product was purified by recrystallization from ethyl acetate (red violet crystals, yield: 130 mg, 10.9%).
¹H-NMR (400 MHz, DMSO-d₆, δ = 2.49 ppm): 11.58 (s, 1H, N'H), 10,74 (s, 1H, NH), 8.84 (dd, 1 H, ³J_{H,H} = 7.9 Hz, ⁴J_{H,H} = 1.7 Hz, 4-CH), 8.50 (dd, 1 H, ³J_{H,H} = 4.8 Hz, ⁴J_{H,H} = 1.7 Hz, 6'-CH), 8.15 - 8.11 (m, 2H, 4'-CH and 6-CH), 7.15-7.07 (m, 2H, 5-CH and 5'-CH).
¹³C-{¹H}-NMR (150 MHz, DMSO-d₆, δ = 39.5 ppm): 186.3 (C3'), 170.5 (C2), 163.4 (C7a'), 155.8 (C6'), 155.4 (C7a), 147.8 (C6), 138.7 (C2'), 133.8 (C4'), 131.5 (C4), 118.2 (C5), 117.9 (C5'), 115.5 (C3a'), 112.9 (C3a), 105.7 (C3).

### Example 4: 3-Formyl-7-azaindole

Hexamethylenetetramine (1.79 g, 12.8 mmol) was added at once to 7-azaindole (1.0 g, 8.5 mmol) in acetic acid (33%, 15 mL). The mixture was refluxed for 4 h, poured into water (25 mL) and cooled to 4°C. The fine crystalline precipitate was filtered off, washed with water and dried in vacuo. Yield: 674.3 mg (4.6 mmol, 54 %).
¹H-NMR (400,13 MHz, DMSO-d₆, δ = 2,49 ppm): 12,68 (s, 1H, NH), 9,91 (s, 1H, 3-CHO), 8,46 (s, 1 H, H2), 8,39 (dd, 1 H, ³J_{H,H} = 7,8 Hz, ⁴J_{H,H} = 1,6 Hz, H4), 8,36 (dd, 1 H, ³J_{H,H} = 4,7 Hz, ⁴J_{H,H} = 1,6 Hz, H6), 7,27 (dd, 1 H, ³J_{H5,H4} = 7,8 Hz, ³J_{H5,H6} = 4,7 Hz, H5)

### Example 5: 1-Acetyl-3-formyl-7-azaindole

Triethylamine (350 µL) and DMAP (25 mg) were added to a suspension of 3-formyl-7-azaindole (310 mg, 2.1 mmol) in acetic anhydride (10 mL) and stirred for 3h at room temperature. The white precipitate was filtered off, washed with water till neutral, and dried in an exsiccator over KOH. Additional product was isolated by keeping the mother liquor at 4°C. Yield: 300.0 mg (1.6 mmol, 76 %).
¹H-NMR (400,13 MHz, DMSO-d₆, δ = 2,49 ppm): 10,08 (s, 1H, 3-CHO), 8,95 (s, 1 H, H2), 8,52 (dd, 1 H, ³J_{H,H} = 4,8 Hz, ⁴J_{H,H} = 1,7 Hz, H6), 8,48 (dd, 1 H, ³J_{H,H} = 7,8 Hz, ⁴J_{H,H} = 1,7 Hz, H4), 7,46 (dt, ³J_{H5,H4} = 7,8 Hz, ³J_{H5,H6} = 4,8 Hz, H5), 3,03 (s, 3H, 1-CO-CH₃).

### Example 6: 1-Acetyl-7-aza-3-indolinone

865 mg (7.6 mmol) of solid 3-chloroperoxybenzoic acid (70%) were added at 0°C to 1-acetyl-3-formyl-7-azaindol (500 mg, 2.7 mmol) in dichloromethane (21 mL) and stirred for 2 h at 0°C and for 24 h at room temperature. The mixture was poured into 35 mL of diluted sodium sulphite (10%). The layers were separated and the aqueous layer was extracted two times with 30 mL of dichloromethane each. The combined organic layers were dried over magnesium sulphate, concentrated and stored at -20°C to yield the colourless crystalline product. It was separated by suction filtration, washed with ethyl acetate and dried in vacuo. Yield: 82.7 mg (0.5 mmol, 17 %).
¹H-NMR (400,13 MHz, DMSO-d₆, δ = 2,49 ppm): 8,66 (s, 1 H, 3-OH), 8,49 (dd, 1 H, ³J_{H,H} = 4,8 Hz, ⁴J_{H,H} = 1,5 Hz, H6), 8,08 (s, 1 H, H2), 8,05 (dd, 1 H, ³J_{H,H} = 7,9 Hz, ⁴J_{H,H} = 1,7 Hz, H4), 7,39 (dd, 1 H, ³J_{H5,H4} = 7,7 Hz, ³J_{H5,H6} = 4,8 Hz, H5), 2,98 (s, 3H, 1-CO-CH₃)

### Example 7: 5-Methyl-7'-azaindirubin

Under argon atmosphere, 1-acetyl-7-aza-3-indolinone (75 mg, 0.4 mmol) was added to a suspension of 5-methylisatin (80 mg. 0.5 mmol) and sodium carbonate (106 mg, 1.0 mmol) in degassed methanol (10 mL). The mixture was stirred for 24 h, the product formed was separated by filtration, washed with little methanol and water and dried over KOH. Yield: 76,4 mg (0.3 mmol, 65 %).
¹H-NMR (400,13 MHz, DMSO-d₆, δ = 2,49 ppm): 10,91 (s, 1H, N1H), 10,78 (s, 1H, N1'H), 8,53 (s, 1 H, H4), 8,48 (d, 1 H, ³J_{H,H} = 3,9 Hz, H6'), 8,09 (d, 1 H, ³J_{H,H} = 6,7 Hz, H4'), 7,05-7,15 (m, 2H, H5' und H6), 6,81 (d, 1H, ³J_{H,H} = 7,4 Hz, H7), 2,31 (s, 3H, CH₃).
¹³C-{¹H}-NMR (150,9 MHz, DMSO-d₆, δ = 39,7 ppm): 186,3 (C3'), 171,3 (C2), 163,1 (C7a'), 155,9 (C6'), 139,4 (C7a), 137,7 (C2'), 133,8 (C4'), 131,0 (C4), 130,5 (C5), 125,5 (C6), 121,2 (C3a), 118,0 (C5'), 113,1 (C3a'), 109,9 (C7), 108,8 (C3), 21,3(CH₃).

### Example 8: 2-(Trimethylacetylamino)-3-methylpyridine

Trimethylacetyl chloride (13.26 g, 0.11 mol) in methylene chloride (20 mL) was slowly added to a solution of 2-amino-3-methylpyridine (10.81 g, 0.1 mol) and triethylamine (12.63 g, 0.12 mol) in 150 ml of methylene chloride at 0°C. The mixture was stirred in an ice bath for 1 h and further at room temperature for 2 h. Subsequently, the mixture was washed three times with an equal volume of aqueous sodium hydrogen carbonate (5%). The organic layer was dried with magnesium sulphate and concentrated to yield a colorless solid (14.52 g, 0.076 mol, 76 %).
¹H-NMR (400 MHz, DMSO-d₆, δ = 2.49 ppm): 9.55 (s, 1H, NH), 8.25 (d, 1 H, ³J_{H,H} = 4.7 Hz, 6-CH), 7.64 (d, 1 H, ³J_{H,H} = 7.4 Hz, 4-CH), 7.19 (dd, 1 H, ³J_{H,H} = 7.4 Hz, ⁴J_{H,H} = 4.7 Hz, 5-CH), 2.10 (s, 3H, 3-CH₃),1.22 (s, 9H, Pv-CH₃).
¹³C-{¹H}-NMR (150 MHz, DMSO-d₆, δ = 39.5 ppm): 176.5 (CO), 150.6 (C2), 145.7 (C6), 139.0 (C4), 129.9 (C3), 121.9 (C5), 38.7 (Pv-C), 27.3 (Pv-CH₃), 17.4 (3-CH₃).

### Example 9: 2-Amino-3-pyridineacetic acid

50 mL of n-butyllithium in hexane (1.6 M, 80 mmol) were slowly added to a solution of 2-(trimethylacetylamino)-3-methylpyridine (6.15 g, 32 mmol) in dry THF (90 mL) at -78 °C in a period of 1 h. The mixture was stirred at -78 °C for 1h and at -20 °C for 3 h. Powdered dry ice was added to this suspension at -78 °C. The mixture was allowed to warm up to room temperature, diluted with water and acidified with 4 N HCl to pH = 5. The mixture was extracted three times with an equal volume of ethyl acetate and the organic layer was dried over magnesium sulphate. After removal of the solvent the residue was dissolved in 6 N HCl and refluxed overnight. Neutralization with 6 N NaOH (pH = 5) produced a precipitate, that was collected by filtration to afford a colourless solid (2 g, 12.0 mmol, 38 %).
¹H-NMR (400 MHz, DMSO-d₆, δ = 2.49 ppm): 7.83 (dd, 1H, ³J_{H,H} = 4.8 Hz, ⁴J_{H,H} = 1.4 Hz, 6-CH), 7.26 (dd, 1 H, ³J_{H,H} = 7.2 Hz, ⁴J_{H,H} = 1.4 Hz, 4-CH), 6.50 (dd, 1 H, ³J_{H,H} = 7.2 Hz, ⁴J_{H,H} = 5.1 Hz, 5-CH), 5.70, (s, 3H, NH₃⁺), 3.42 (s, 2H, CH₂).

### Example 10: 7-Azaoxindole

The mother liquor of the foregoing example was extracted three times with an equal volume of ethyl acetate and the combined organic phases were dried over sodium sulphate. Removal of the solvent afforded the product as colourless solid.

A suspension of 2-amino-3-pyridineacetic acid (2.0 g, 12.0 mmol) and a catalytic amount of *p*-toluenesulfonic acid in amyl alcohol was refluxed for 48 h. After removal of the solvent from the clear solution, the residue was purified by column chromatography on silica gel using ethyl acetate : hexane (3:1) as eluent to provide a solid (0.5 g, 3.7 mmol, 31 %).
¹H-NMR (400 MHz, DMSO-d₆, δ = 2.49 ppm): 10.96 (s, 1 H, NH), 8.02 (d, 1 H, ³J_{H,H} = 5.3 Hz, 6-CH), 7.52 (d, 1 H, ³J_{H,H} = 7.0 Hz, 4-CH), 6.91 (dd, 1 H, ³J_{H,H} = 7.0 Hz, ⁴J_{H,H} = 5.3Hz, 5-CH), 3.53 (s, 2H, CH₂).
¹³C-{¹H}-NMR (150 MHz, DMSO-d₆, δ = 39.5 ppm): 1765.8 (C2), 1508.2 (C7a), 146.1 (C6), 131.8 (C4), 120.4 (C3a), 117.3 (C5), 35.2 (C3).

### Example 11: 7-Azaisatin

A suspension of 7-azaoxindole (500 mg, 3.7 mmol) and *N*-bromosuccinimide (690.6 mg, 3.9 mmol) in dry DMSO (13 mL) was stirred at 60 °C for 2 h and at 95 °C for 8 h under reduced pressure (100 hPa). The mixture was adjusted to pH 5-6 with aqueous NaHCO₃ (5%) and extracted with ethyl acetate. The solvent was removed and the residue was purified by column chromatography using ethyl acetate : hexane (3:1) as eluent to afford a yellow solid (170 mg, 1.15 mmol, 31 %).
¹H-NMR (400 MHz, DMSO-d₆, δ = 2.49 ppm): 11.6 (s, 1 H, NH), 8.38 (dd, 1 H, ³J_{H,H} = 5.2 Hz, ⁴J_{H,H} = 1.8 Hz, 6-CH), 7.87 (dd, 1 H, ³J_{H,H} = 7.5 Hz, ⁴J_{H,H} = 0.9 Hz, 4-CH), 7.09 (dd, 1 H, ³J_{H,H} = 7.4 Hz, ⁴J_{H,H} = 5.2 Hz, 5-CH).

### Example 12: 7-Azaindirubin

### Basic condensation:

Under argon atmosphere a suspension of indoxyl-3-acetate (200 mg, 1.1 mmol), 7-azaisatin (183 mg, 1.2 mmol) and sodium carbonate (264 mg, 2.5 mmol) in degassed methanol (25 mL) was stirred at room temperature for 5 h, diluted with water and filtered. The solution was extracted with ethyl acetate. After removal of the solvent, the residue was purified by column chromatography on silica gel using ethyl acetate : hexane (1:1) as eluent to get a reddish solid (18.5 mg, 0.07 mmol, 6.4 %).
¹H-NMR (600 MHz, DMSO-d₆, δ = 2.49 ppm): 11.42 (s, 1 H, NH), 11.07 (s, 1 H, N'H), 8.90 (d, 1 H, ³J_{H,H} = 4.4 Hz, 4-CH), 8,10 (d, 1 H, ³J_{H,H} = 2.4 Hz, 6-CH), 7.66 (d, 1 H, ³J_{H,H} = 5.0, 4'-CH), 7,59 (t, 1 H, ³J_{H,H} = 5.5 Hz, 6-CH), 7,40 (d, 1 H, ³J_{H,H} = 5,3 Hz, 7'-CH), 7,03-7.06 (m, 2H, ³J_{H,H} = 7,6 Hz, 5 and 5'-CH).

### Acid condensation:

7-Azaisatin (67.1 mg, 0.45 mmol) and indoxyl-3-acetate (70 mg, 0.4 mmol) were suspended under argon atmosphere in a mixture of acetic acid (4 mL) and conc. HCl (340 µL). The mixture was refluxed for 3 h and diluted with water (30 ml). The precipitate was filtrated and purified by column chromatography on silica gel using acetyl acetate : hexan (3:1) as eluent to yield a reddish solid (40 mg, 0.15 mmol, 33.8 %).

### Example 13: 7,7'-Diazaindirubin (Method 2)

7-Azaisatin (67.1 mg, 0.45 mmol) and 7-azaindoxyl-3-acetate (70 mg, 0.4 mmol) were suspended under argon atmosphere in acetic acid (4 mL) and conc. HCl (340 µL). The mixture was refluxed for 6 h and diluted with water (30 ml). The precipitate was filtrated, suspended in ethyl acetate (150 ml), and refluxed for 10 min. The hot mixture was filtrated and cooled. After 24 h at -32 °C, the precipitate was collected and dried to get a reddish solid (35 mg, 0.13 mmol, 29.6 %).

### Example 14: 7'-Azaindirubin-3'-oxime

A solution of 7-azaindirubin (275 mg, 1.1 mmol) and hydroxylamine hydrochloride (196.4 mg, 24.4 mmol) in pyridine (7 mL) was refluxed for 4 h. The mixture was diluted with 2 N HCl (50 mL) to yield a reddish solid (230 mg, 0.77 mmol, 70 %).
¹H-NMR (600 MHz, DMSO-d₆, δ = 2.49 ppm): 13.82 (s, 1H, NOH), 11.79 (s, 1H, N'H), 10.90 (s, 1 H, NH), 8.59 (d, 1 H, ³J_{H,H} = 7.6 Hz, 4-CH), 8.47 (d, 1 H, ³J_{H,H} = 6.4 Hz, 6'-CH), 8.29 (d, 1 H, ³J_{H,H}= 5.0, 4'-CH), 7,.18 (dt, 1 H, ³J_{H,H} = 7.2 Hz, ⁴J_{H,H} = 0.8 Hz, 6-CH), 7.09 (dd, 1 H, ³J_{H,H} = 5.0 Hz, ³J_{H,H} = 7.3 Hz, 5'-CH), 6.98 (t, 1 H, ³J_{H,H} = 7.6 Hz, 5-CH), 6.93 (d, 1 H, ³J_{H,H} = 7.6 Hz, 7-CH).
¹³C-{¹H}-NMR (150 MHz, DMSO-d₆, δ = 39.5 ppm): 171.2 (C2), 156.8 (C3'), 150.9 (C6'), 149.3 (C7a'), 143.2 (C7a), 139.0 (C2'), 136.0 (C4'), 127.1 (C4), 123.4 (C6), 122.2 (C3a), 120.9 (C5), 117.8 (C5'), 110.4 (C3a'), 109.4 (C7), 101.0 (C3).

### Example 15: 7'-Azaindirubin-3'-(2-bromoethyl)-oxime ether

A mixture of 7-azaindirubin-3'-oxime (230 mg, 0.83 mmol), 1,2-dibromoethane (1.6 g, 8.3 mmol) and 1,1,3,3-tetramethylguanidine (400 µL) in ethanol (10 mL) was refluxed for 3 h. The mixture was diluted with water (50 mL), filtrated and dried to afford a reddish solid (120 mg, 0.312 mmol, 37.5 %).
¹H-NMR (600 MHz, DMSO-d₆, δ = 2.49 ppm): 11.40 (br, 1H, N1'H), 10.50 (br, 1H, N1 H), 8.49 (d, 1 H, ³J_{H,H}= 8.2 Hz, 4-CH), 8.44 (d, 1 H, ³J_{H,H} = 7.3 Hz, 6'-CH), 8.32 (d, 1 H, ³J_{H,H} = 3.8, 4'-CH), 7.19 (t, 1 H, ³J_{H,H} = 6.7 Hz, 6-CH), 7.09 (t, 1 H, ³J_{H,H} = 6.5 Hz, 5'-CH), 7.01 (t, 1 H, ³J_{H,H} = 7.3 Hz, 5-CH), 6.93 (d, 1 H, ³J_{H,H}= 7.6 Hz, 7-CH), 4.91 (t, 2H, ³J_{H,H} = 5.0 Hz, 1-CH₂), 3.97 (t, 2H, ³J_{H,H} = 5.6 Hz, 2-CH₂).

### Example 16: 7'-Azaindirubin-3'-[2-(4-methylpiperazino)-ethyl]-oxime ether

A suspension of 7'-azaindirubin-3'-(2-bromoethyl)-oxime ether (60 mg, 0.15 mmol) and 1-methylpiperazine (500 mg, 5 mmol) in dry DMF (10 mL) was stirred at room temperature for 24 h. The mixture was diluted with water (50 mL) and extracted with ethyl acetate. The solvent was removed in vacuo and the residue was purified by column chromatography using ethanol as eluent to yield a reddish solid (20 mg, 0.049 mmol, 33.0 %).
¹H-NMR (600 MHz, DMSO-d₆, δ = 2.49 ppm): 11.07 (s, 1H, N1'H), 11.03 (s, 1H, N1 H), 7.56 (d, 1 H, ³J_{H,H} = 7.6 Hz, 4-CH), 8.41 (d, 1 H, ³J_{H,H} = 6.6 Hz, 6'-CH), 8.31 (d, 1 H, ³J_{H,H} = 3.6, 4'-CH), 7.19 (t, 1 H, ³J_{H,H} = 6.8 Hz, 6-CH), 7.08 (t, 1 H, ³J_{H,H} = 5.3 Hz, 5'-CH), 6.79 (t, 1 H, ³J_{H,H} = 7.9 Hz, 5-CH), 6.74 (d, 1 H, ³J_{H,H} = 7.6 Hz, 7-CH), 4.69 (t, 2H, ³J_{H,H} = 5.9 Hz, Et1-CH₂), 2.96 (t, 2H, ³J_{H,H} = 5.9 Hz, Et2-CH₂), 2.49 (br, 4H, Pip2 and Pip6-CH₂), 2.30 (br, 4H, Pip3 and Pip5-CH₂), 2.12 (s, 3H, CH₃).

## Claims

1. An azaindirubin compound, selected from 7'-azaindirubin, and 7,7'-diaza-indirubin derivatives, having the following general formulae: 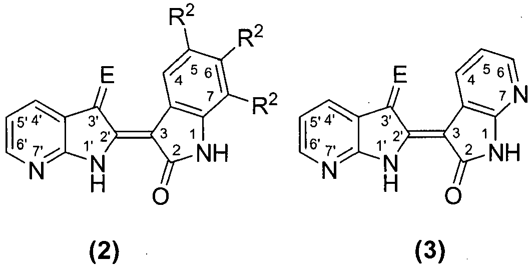 wherein
R² stands for hydrogen, C₁-C₄ alkyl, halogen, hydroxy, C₁-C₄ alkoxy, mercapto, carboxy, or an amino group NR⁴R⁵, or a carboxamido group CONR⁴R⁵, wherein R⁴ and R⁵ can be the same or different and represent a hydrogen atom, a straight-chain or branched-chain C₁-C₄ alkyl group,
which can additionally carry one or more halogen, hydroxy and/or amino groups, a substituted or unsubstituted aryl group which can comprise one or more heteroatoms, or the acyl groups -COM, -COOM, -CH₂COOM wherein M is hydrogen, a straight-chain or branched-chain alkyl group having 1 to 4 carbon atoms which can additionally carry one or more hydroxy and/or amino groups, or an aryl group which can comprise one or more heteroatoms and can be substituted with one or more halogen atoms, one or more alkyl groups or one or more alkoxy groups;
or C₃-C₇ cycloalkyl, C₃-C₇ cycloalkenyl,
that is optionally interrupted by one or more oxygen atoms and/or can additionally carry one or more C₁-C₄ alkyl, halogen, hydroxy and/or amino groups, a substituted or unsubstituted aryl group which can comprise one or more heteroatoms, or the acyl groups -COM, -COOM, -CH₂COOM with the definition for M above,
or R⁴ and R⁵ together with the nitrogen atom of the amino group form a ring,
which can contain one or more heteroatoms and have 2 to 8, optionally substituted, CH₂ groups which can additionally carry one or more C₁-C₄ alkyl, C₃-C₇ cycloalkyl, halogen, hydroxy and/or amino groups, a substituted or unsubstituted aryl group which can comprise one or more heteroatoms, or the acyl groups -COM, -COOM, -CH₂COOM with the definition for M above;
E in formulae (2) and (3) means an oxygen atom, an oxime group [NOH], or an oxime ether group [NOR³], with R³ representing an alkyl chain with formula (CH₂)ₙR', in which n stands for 2-6 and R' stands for an amino group NR⁴R⁵, wherein R⁴ and R⁵ can be the same or different and represent a hydrogen atom, a straight-chain or branched-chain C₁-C₄ alkyl group,
which can additionally carry one or more halogen, hydroxy and/or amino groups, a substituted or unsubstituted aryl group which can comprise one or more heteroatoms, or the acyl groups -COM, -COOM, -CH₂COOM wherein M is hydrogen, a straight-chain or branched-chain alkyl group having 1 to 4 carbon atoms which can additionally carry one or more hydroxy and/or amino groups, or an aryl group which can comprise one or more heteroatoms and can be substituted with one or more halogen atoms, one or more alkyl groups or one or more alkoxy groups;
or C₃-C₇ cycloalkyl, C₃-C₇ cycloalkenyl,
that is optionally interrupted by one or more oxygen atoms and/or can additionally carry one or more C₁-C₄ alkyl, halogen, hydroxy and/or amino groups, a substituted or unsubstituted aryl group which can comprise one or more heteroatoms, or the acyl groups -COM, -COOM, -CH₂COOM with the definition for M above,
or R⁴ and R⁵ together with the nitrogen atom of the amino group form a ring,
which can contain one or more heteroatoms and have 2 to 8, optionally substituted, CH₂ groups which can additionally carry one or more C₁-C₄ alkyl, C₃-C₇ cycloalkyl, halogen, hydroxy and/or amino groups, a substituted or unsubstituted aryl group which can comprise one or more heteroatoms, or the acyl groups -COM, -COOM, -CH₂COOM with the definition for M above.

2. The azaindirubin compound according to claim 1, which is in the form of a physiologically compatible salt formed with organic or inorganic acids, selected from hydrochloric acid, sulfuric acid, phosphoric acid, citric acid, tartaric acid, lactic acid, succinic acid, acetic acid, malic acid, or fumaric acid.

3. The azaindirubin compound according to claim 1 or 2 wherein E stands for C=O or for C=NO(CH₂)ₙNR⁴R⁵, wherein n is 2, and NR⁴R⁵ represents an N-methylpiperazino substituent.

4. A method of producing an azaindirubin compound according to claim 1, 2 or 3 by condensation of a corresponding 7-azaisatin with 3-indoxyl-3-acetates, 7-aza-3-indoxyl-3-acetate, 1-acetylindoxyls, 1,3-diacetylindoxyls or 1-acetyl-7-azaindoxyl, using basic or acid reaction conditions.

5. The method according to claim 4, wherein 7-aza-3-indoxyl-3-acetate is produced by reaction of 7-azaindole with thallium(III)-acetate.

6. The method according to claim 4, wherein 1-acetyl-7-azaindoxyl is obtained by starting with 7-azaindole via formylation, acetylation and Baeyer-Villiger-oxidation.

7. The method according to claim 4, wherein 7-azaisatin is obtained by oxidation of 7-azaoxindole using NBS/DMSO.

8. The method according to claim 4, wherein 7,7'-diazaindirubin is obtained by reaction of 7-azaindole with thallium(III)-acetate.

9. A pharmaceutical formulation comprising at least one of the compounds according to claim 1, 2 or 3.

10. Compound according to claim 1, 2 or 3 or the pharmaceutical formulation according to claim 9 for use in treating cancer, neurodegenerative diseases, bipolar disorders, inflammatory and infectious diseases, including viral diseases.

## Patentansprüche

1. Azaindirubinverbindung, ausgewählt aus 7'-Azaindirubin- und 7,7'-Diaza-indirubin-Derivaten mit den folgenden allgemeinen Formeln: 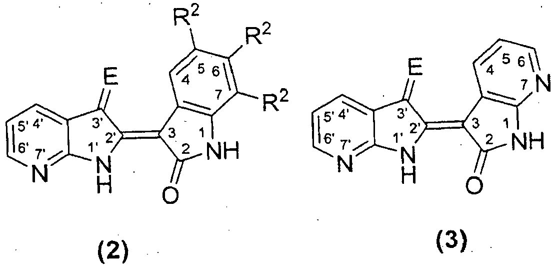 worin
R² für Wasserstoff, C₁-C₄ Alkyl, Halogen, Hydroxy, C₁-C₄ Alkoxy, Mercapto, Carboxy oder eine Aminogruppe NR⁴R⁵ oder eine Carboxamidogruppe CONR⁴R⁵ steht, wobei R⁴ und R⁵ gleich oder unterschiedlich sein können und ein Wasserstoffatom, eine geradkettige oder verzweigtkettige C₁-C₄ Alkylgruppe,
welche zusätzlich ein oder mehrere Halogen-, Hydroxy- und/oder Aminogruppen, eine substituierte oder unsubstituierte Arylgruppe, welche ein oder mehrere Heteroatome umfassen kann, oder die Acylgruppen COM, - COOM, -CH₂COOM, wobei M Wasserstoff, eine geradkettige oder verzweigtkettige Alkylgruppe mit 1 bis 4 Kohlenstoffatomen, welche zusätzlich ein oder mehrere Hydroxy- und/oder Aminogruppen tragen kann, oder eine Arylgruppe, welche ein oder mehrere Heteroatome umfassen kann und mit einem oder mehreren Halogenatomen, einer oder mehreren Alkylgruppen oder einer oder mehreren Alkoxygruppen substituiert sein kann, tragen können;
oder C₃-C₇ Cycloalkyl, C₃-C₇ Cycloalkenyl darstellen,
welche gegebenenfalls mit einem oder mehreren Sauerstoffatomen unterbrochen sein kann und/oder zusätzlich ein oder mehrere C₁-C₄ Alkyl-, Halogen-, Hydroxy-, und/oder Aminogruppen, eine substituierte oder unsubstituierte Arylgruppe, welche ein oder mehrere Heteroatome umfassen kann, oder die Acylgruppen -COM, -COOM, -CH₂COOM, mit der Definition für M vorstehend, tragen kann,
oder R⁴ und R⁵ zusammen mit dem Stickstoffatom der Aminogruppe einen Ring bilden, welcher ein oder mehrere Heteroatome tragen kann und 2 bis 8, gegebenenfalls substituierte, CH₂ Gruppen aufweist, welche zusätzlich ein oder mehrere C₁-C₄ Alkyl-, C₃-C₇ Cycloalkyl-, Halogen-, Hydroxy- und/oder Aminogruppen, eine substituierte oder unsubstituierte Arylgruppe, welche ein oder mehrere Heteroatome umfassen kann, oder die Acylgruppen -COM, -COOM, -CH₂COOM, mit der Definition für M vorstehend, tragen kann,
E in den Formeln (2) und (3) ein Sauerstoffatom, eine Oximgruppe [NOH] oder eine Oximethergruppe [NOR³] bedeutet, wobei R³ eine Alkylkette mit der Formel (CH₂)ₙR' darstellt, worin n für 2-6 steht und R' für eine Aminogruppe NR⁴R⁵ steht, wobei R⁴ und R⁵ gleich oder unterschiedlich sein können und ein Wasserstoffatom, eine geradkettige oder verzweigtkettige C₁-C₄ Alkylgruppe,
welche zusätzlich ein oder mehrere Halogen-, Hydroxy- und/oder Aminogruppen, eine substituierte oder unsubstituierte Arylgruppe, welche ein oder mehrere Heteroatome umfassen kann, oder die Acylgruppen COM, - COOM, -CH₂COOM, wobei M Wasserstoff, eine geradkettige oder verzweigtkettige Alkylgruppe mit 1 bis 4 Kohlenstoffatomen, welche zusätzlich ein oder mehrere Hydroxy- und/oder Aminogruppen tragen kann, oder eine Arylgruppe, welche ein oder mehrere Heteroatome umfassen kann und mit einem oder mehreren Halogenatomen, einer oder mehreren Alkylgruppen oder einer oder mehreren Alkoxygruppen substituiert sein kann, tragen können;
oder C₃-C₇ Cycloalkyl, C₃-C₇ Cycloalkenyl darstellen,
welche gegebenenfalls mit einem oder mehreren Sauerstoffatomen unterbrochen sein kann und/oder zusätzlich ein oder mehrere C₁-C₄ Alkyl-, Halogen-, Hydroxy-, und/oder Aminogruppen, eine substituierte oder unsubstituierte Arylgruppe, welche ein oder mehrere Heteroatome umfassen kann, oder die Acylgruppen -COM, -COOM, -CH₂COOM, mit der Definition für M vorstehend, tragen kann,
oder R⁴ und R⁵ zusammen mit dem Stickstoffatom der Aminogruppe einen Ring bilden, welcher ein oder mehrere Heteroatome tragen kann und 2 bis 8, gegebenenfalls substituierte, CH₂ Gruppen aufweist, welche zusätzlich ein oder mehrere C₁-C₄ Alkyl-, C₃-C₇ Cycloalkyl-, Halogen-, Hydroxy- und/oder Aminogruppen, eine substituierte oder unsubstituierte Arylgruppe, welche ein oder mehrere Heteroatome umfassen kann, oder die Acylgruppen -COM, -COOM, -CH₂COOM, mit der Definition für M vorstehend, tragen kann.

2. Azaindirubinverbindung gemäß Anspruch 1, welche in der Form eines physiologisch verträglichen Salzes, gebildet mit organischen oder anorganischen Säuren, ausgewählt aus Salzsäure, Schwefelsäure, Phosphorsäure, Zitronensäure, Weinsäure, Milchsäure, Bernsteinsäure, Essigsäure, Äpfelsäure oder Fumarsäure, vorliegt.

3. Azaindirubinverbindung gemäß Anspruch 1 oder 2, wobei E für C=O oder für C=NO(CH₂)ₙNR⁴R⁵ steht, wobei n 2 ist und NR⁴R⁵ einen N-Methylpiperazino-Substituenten darstellt.

4. Verfahren zur Herstellung einer Azaindirubinverbindung gemäß Anspruch 1, 2 oder 3 durch Kondensation eines entsprechenden 7-Azaisatins mit 3-Indoxyl-3-acetaten, 7-Aza-3-indoxyl-3-acetat, 1-Acetylindoxylen, 1,3-Diacetylindoxylen oder 1-Acetyl-7-azaindoxyl unter Einsatz basischer oder saurer Reaktionsbedingungen.

5. Verfahren gemäß Anspruch 4, wobei 7-Aza-3-indoxyl-3-acetat durch Umsetzung von 7-Azaindol mit Thallium(III)acetat erzeugt wird.

6. Verfahren gemäß Anspruch 4, wobei 1-Acetyl-7-azaindoxyl durch Starten mit 7-Azaindol über Formylierung, Acetylierung und Baeyer-Villiger-Oxidation erhalten wird.

7. Verfahren gemäß Anspruch 4, wobei 7-Azaisatin durch Oxidation von 7-Azaoxindol unter Verwendung von NBS/DMSO erhalten wird.

8. Verfahren gemäß Anspruch 4, wobei 7,7'-Diazaindirubin durch Umsetzung von 7-Azaindol mit Thallium(III)acetat erhalten wird.

9. Pharmazeutische Formulierung, umfassend mindestens eine der Verbindungen gemäß Anspruch 1, 2 oder 3.

10. Verbindung gemäß Anspruch 1, 2 oder 3 oder die pharmazeutische Formulierung gemäß Anspruch 9 zur Verwendung in der Behandlung von Krebs, neurodegenerativen Krankheiten, bipolaren Störungen, entzündlichen und infektiösen Krankheiten, einschließlich viraler Krankheiten.

## Revendications

1. Composé d'azaindirubine, choisi parmi des dérivés de la 7'-azaindirubine, et de la 7,7'-diaza-indirubine, ayant les formules générales suivantes : 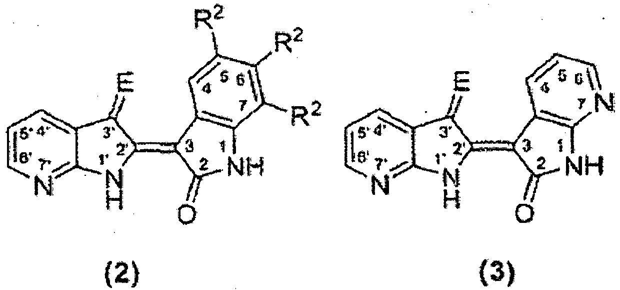 dans lesquelles
R² représente un atome d'hydrogène, un groupe alkyle en C₁ à C₄, halogéno, hydroxy, alcoxy en C₁ à C₄, mercapto, carboxy, ou un groupe amino NR⁴R⁵, ou un groupe carboxamido CONR⁴R⁵, où R⁴ et R⁵ peuvent être identiques ou différents et représentent un atome d'hydrogène, un groupe alkyle en C₁ à C₄ à chaîne linéaire ou à chaîne ramifiée,
qui peut porter en outre un ou plusieurs atomes d'halogène, groupes hydroxy et/ou amino, un groupe aryle substitué ou non substitué qui peut comprendre un ou plusieurs hétéroatome, ou les groupes acyle -COM, - COOM, -CH₂COOM où M représente un atome d'hydrogène, un groupe alkyle à chaîne linéaire ou à chaîne ramifiée, comportant 1 à 4 atomes de carbone qui peut porter en outre un ou plusieurs groupes hydroxy et/ou amino, ou un groupe aryle qui peut comprendre un ou plusieurs hétéroatome et peut être substitué par un ou plusieurs atomes d'halogène, un ou plusieurs groupes alkyle ou un ou plusieurs groupes alcoxy ;
ou un groupe cycloalkyle en C₃ à C₇, cycloalcényle en C₃ à C₇,
qui est éventuellement interrompu par un ou plusieurs atomes d'oxygène et/ou peut porter en outre un ou plusieurs groupes alkyle en C₁ à C₄, halogéno, hydroxy et/ou amino, un groupe aryle substitué ou non substitué qui peut comprendre un ou plusieurs hétéroatomes, ou les groupes acyle -COM, -COOM, -CH₂COOM avec la définition pour M ci-dessus,
ou R⁴ et R⁵ conjointement avec l'atome d'azote du groupe amino forment un cycle,
qui peut contenir un ou plusieurs hétéroatomes et comporte 2 à 8 groupes CH₂, éventuellement substitués, qui peuvent porter en outre un ou plusieurs groupes alkyle en C₁ à C₄, cycloalkyle en C₃ à C₇, halogéno, hydroxy et/ou amino, un groupe aryle substitué ou non substitué qui peut comprendre un ou plusieurs hétéroatomes, ou les groupes acyle -COM, -COOM,-CH₂COOM avec la définition pour M ci-dessus ;
É dans les formules (2) et (3) signifie un atome d'oxygène, un groupe oxime [NOH], ou un groupe éther d'oxime [NOR³], avec R³ représentant une chaîne alkyle de formule (CH₂)ₙR', dans laquelle n vaut de 2 à 6 et R' représente un groupe amino NR⁴R⁵, où R⁴ et R⁵ peuvent être identiques ou différents et représentent un atome d'hydrogène, un groupe alkyle en C₁ à C₄ à chaîne linéaire ou à chaîne ramifiée,
qui peut porter en outre un ou plusieurs atomes d'halogène, groupes hydroxy et/ou amino, un groupe aryle substitué ou non substitué qui peut comprendre un ou plusieurs hétéroatomes, ou les groupes acyle -COM,-COOM, -CH₂COOM où M représente un atome d'hydrogène, un groupe alkyle à chaîne linéaire ou à chaîne ramifiée comportant de 1 à 4 atomes de carbone qui peut porter en outre un ou plusieurs groupes hydroxy et/ou amino, ou un groupe aryle qui peut comprendre un ou plusieurs hétéroatome et peut être substitué par un ou plusieurs atomes d'halogène, un ou plusieurs groupes alkyle ou un ou plusieurs groupes alcoxy ;
ou cycloalkyle en C₃ à C₇, cycloalcényle en C₃ à C₇,
qui est éventuellement interrompu par un ou plusieurs atomes d'oxygène et/ou peut éventuellement porter un ou plusieurs groupes alkyle en C₁ à C₄, halogéno, hydroxy et/ou amino, un groupe aryle substitué ou non substitué qui peut comprendre un ou plusieurs hétéroatomes, ou les groupes acyle -COM, -COOM, -CH₂COOM avec la définition pour M ci-dessus,
ou R⁴ et R⁵ conjointement avec l'atome d'azote du groupe amino forment un cycle,
qui peut contenir un ou plusieurs hétéroatomes et comporter de 2 à 8 groupes CH₂, éventuellement substitués, qui peuvent porter en outre un ou plusieurs groupes alkyle en C₁ à C₄, cycloalkyle en C₃ à C₇, halogéno, hydroxy et/ou amino, un groupe aryle substitué ou non substitué qui peut comprendre un ou plusieurs hétéroatomes, ou les groupes acyle -COM,-COOM, -CH₂COOM avec la définition pour M ci-dessus.

2. Composé d'azaindirubine selon la revendication 1, qui est sous la forme d'un sel physiologiquement compatible formé avec des acides organiques ou inorganiques, choisis parmi l'acide chlorhydrique, l'acide sulfurique, l'acide phosphorique, l'acide citrique, l'acide tartrique, l'acide lactique, l'acide succinique, l'acide acétique, l'acide malique, ou l'acide fumarique.

3. Composé d'azaindirubine selon la revendication 1 ou 2, dans lequel E représente C=O ou C=NO(CH₂)ₙNR⁴R⁵, où n vaut 2, et NR⁴R⁵ représente un substituant N-méthylpipérazino.

4. Procédé de production d'un composé d'azaindirubine selon la revendication 1, 2 ou 3 par condensation de 7-azaisatine avec des 3-indoxyl-3-acétates, le 7-aza-3-indoxyl-3-acétate, des 1-acétyl-indoxyles, des 1,3-diacétylindoxyles ou le 1-acétyl-7-azaindoxyle, en utilisant des conditions réactionnelles basiques ou acides.

5. Procédé selon la revendication 4, dans lequel le 7-aza-3-indoxyl-3-acétate est produit par réaction de 7-azaindole avec de l'acétate de thallium(III).

6. Procédé selon la revendication 4, dans lequel le 1-acétyl-7-azaindoxyle est obtenu en démarrant avec du 7-azaindole par l'intermédiaire d'une formylation, d'une acétylation et d'une oxydation de Baeyer-Villiger.

7. Procédé selon la revendication 4, dans lequel la 7-azaisatine est obtenue par oxydation du 7-azaoxindole en utilisant du NBS/DMSO.

8. Procédé selon la revendication 4, dans lequel la 7,7'-diazaindirubine est obtenue par réaction du 7-azaindole avec de l'acétate de thallium(III).

9. Formulation pharmaceutique comprenant au moins l'un des composés selon la revendication 1, 2 ou 3.

10. Composé selon la revendication 1, 2 ou 3 ou composition pharmaceutique selon la revendication 9 pour une utilisation dans le traitement du cancer, de maladies neurodégénératives, de troubles bipolaires, de maladies inflammatoires et infectieuses, y compris des maladies virales.
